# FASCICULE DE BREVET EUROPEEN

(11) **EP 0 949 897 B1**
(45) Date de publication et mention de la délivrance du brevet: **23.05.2001**
(21) Numéro de dépôt: 97948996.0
(22) Date de dépôt: 03.12.1997
(51) Int. Cl.: A61K 7/13, C07D 311/60

(54) **UTILISATION DE COMPOSES DU TYPE FLAVYLIUM NON SUBSTITUES EN POSITION 3 EN TEINTURE DES FIBRES KERATINIQUES ET COMPOSITIONS LES CONTENANT**
VERWENDUNG VON IN POSITION 3 UNSUBSTITUIERTEN VERBINDUNGEN DES FLAVYLIUM-TYPS ZUR FÄRBUNG VON KERATINISCHEN FASERN UND DIESE ENTHALTENDE ZUBEREITUNGEN
USE OF FLAVYLIUM TYPE COMPOUNDS NON-SUBSTITUTED IN POSITION 3 FOR DYEING KERATINOUS FIBRES AND COMPOSITIONS CONTAINING THEM

(30) Priorité: 23.12.1996 FR 9615890
(43) Date de publication de la demande: 20.10.1999
(73) Titulaire: L'OREAL, 75008 Paris (FR)
(72) Inventeur: DARMENTON, Patrick, F-92340 Bourg la Reine (FR); PHILIPPE, Michel, F-91320 Wissous (FR)
(74) Mandataire: Miszputen, Laurent
(86) Numéro de dépôt international: FR9702196
(87) Numéro de publication internationale: WO9827940

(56) Documents cités:
- EP-A- 0 024 731
- WO-A-94/06787
- WO-A-96/37558
- GB-A- 2 009 159
- US-A- 3 266 903
- US-A- 4 208 434

## Description

L'invention concerne l'utilisation de composés du type flavylium, non substitués en position 3, et substitués par au moins un radical hydroxyle ou alcoxy, et notamment l'Apigénidine, à titre de colorants, dans des, ou pour la fabrication de, compositions destinées à la teinture des fibres kératiniques, en particulier des fibres kératiniques humaines telles que les cheveux.

Bien des composés comportant un noyau flavylium (F) : 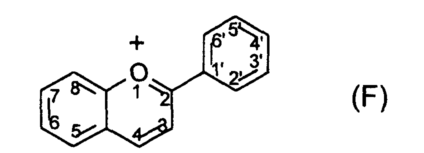 constituent la structure de base des colorants naturels qui sont responsables de la plupart des teintes rouges, bleues ou violettes, des fleurs ou des fruits du monde entier, et que l'on connaît communément sous l'appellation "anthocyanes", ou encore "anthocyanidines", lorsqu'il s'agit de la forme aglycone des anthocyanes.

La plupart des anthocyanes et anthocyanidines comportent respectivement un radical glycoside ou hydroxyle en position 3 du noyau flavylium.

Ces anthocyanes et anthocyanidines ont été autrefois largement utilisés comme colorants naturels dans la teinture des textiles. Leur utilisation pour cette application fut ensuite abandonnée en raison du manque de solidité des teintures obtenues, à la lumière et aux lavages.
Il est bien connu, voir à ce propos le brevet US- 3 266 903, que l'instabilité à la lumière de ces colorants naturels est dépendante de la nature du substituant en position 3 du noyau flavylium. Quand la position 3 du noyau flavylium est occupée par un sucre, comme c'est le cas des anthocyanes, ou par un radical hydroxyle, comme c'est le cas de leur forme aglycone, les anthocyanidines, les colorations sont instables. Ainsi, dans l'industrie alimentaire, il a été préconisé de prévenir la décoloration, engendrée par la lumière, des compositions contenant ces colorants anthocyaniques et anthocyanidiniques peu stables, en les photostabilisant par association à des flavonols (EP- 24 731 A1), ou à des dérivés d'acide ascorbique (US- 4 208 434).

Par ailleurs, dans le domaine de la teinture capillaire, on recherche des colorants directs qui, sans l'apport d'agent oxydant, sont capables de modifier par eux-mêmes la nuance naturelle des cheveux, de façon temporaire.
Dans cette application, les colorants doivent satisfaire à un certain nombre de critères : être le plus inoffensif possible, c'est-à-dire présenter une innocuité acceptable, et engendrer des colorations reproductibles et suffisamment résistantes, en particulier, à la lumière, aux lavages et aux intempéries.
En outre, ces colorants, comme cela est bien connu, sont utilisés en mélanges, afin d'obtenir des nuances naturelles.
Or dans ce domaine, les colorants dits naturels tels que le Henné, n'engendrent que des gammes de nuances voisines du blond. Pour élargir la palette de teintes, il y a lieu de trouver d'autres colorants naturels, dont les nuances sont proches des couleurs fondamentales, et qui, mélangés notamment au Henné, pourront répondre aux attentes des consommateurs.

C'est après d'importantes recherches menées sur la question, que la Demanderesse vient maintenant de découvrir, de façon totalement inattendue et surprenante, des composés du type flavylium non substitués en position 3 et substitués par au moins un radical ou alcoxy, qui permettent de teindre intensément, et sans oxydant, les fibres kératiniques, et en particulier les fibres kératiniques humaines telles que les cheveux, en des nuances particulièrement bien stables à la lumière et aux shampooings.

On a constaté en outre, que les teintures capillaires obtenues à l'aide de ces composés préservaient l'état du cheveu, étaient reproductibles, et présentaient une excellente innocuité.

Cette découverte est à la base de la présente invention.

La présente invention a ainsi pour objet l'utilisation de composés du type flavylium, non substitués en position 3, et substitués par au moins un radical hydroxyle ou alcoxy, sous forme pure ou d'extraits végétaux les contenant, à titre de colorants, dans une, ou pour la préparation d'une, composition destinée à la teinture des fibres kératiniques, en particulier des compositions cosmétiques destinées à la teinture directe des fibres kératiniques humaines telles que les cheveux.

Parmi ces composés du type flavylium, on utilise préférentiellement les composés substitués en position 4' par un radical hydroxy, alcoxy ou hydroxyalkyle, sous forme pure ou sous forme d'extraits végétaux les contenant.

On utilise encore plus particulièrement les composés du type flavylium tels que décrits ci-devant et de formule (I) suivante : 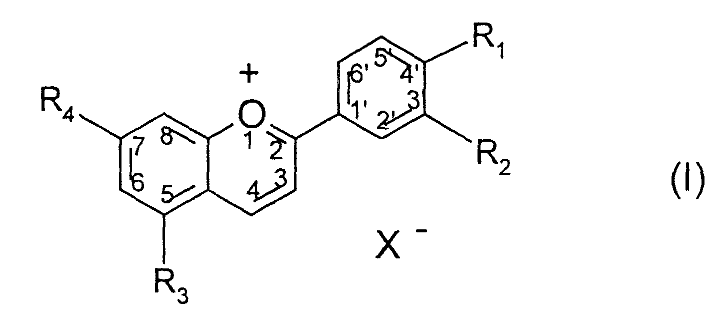 formule (I) dans laquelle :
- **R**_{**1**} désigne un radical OH ou alcoxy en C₁-C₈, linéaire ou ramifié, saturé ou insaturé,
- **R**_{**2**}**, R**_{**3**}**, R**_{**4**}**,** identiques ou différents, désignent H ou R₁, étant entendu qu'au moins un des radicaux R₁ à R₄ désigne OH,
- X⁻ est un anion désignant de préférence un halogène ou un radical : 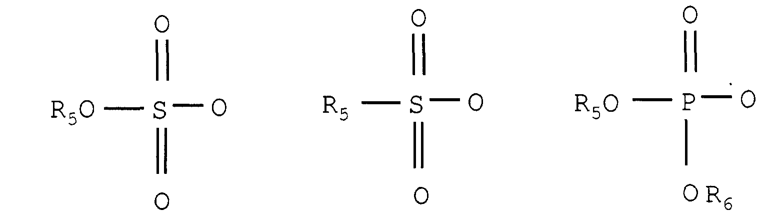 dans lesquels R₅ et R₆ sont des radicaux alkyles en C₁-C₈, linéaires ou ramifiés, saturés ou insaturés,
   sous forme pure ou d'extraits végétaux les contenant.

Un autre objet de l'invention est une composition destinée à la teinture des fibres kératiniques, en particulier une composition cosmétique destinée à la teinture directe des fibres kératiniques humaines telles que les cheveux, comprenant, dans un milieu approprié pour la teinture, 0,05 à 20% et de préférence 0,1 à 10% en poids par rapport au poids total de la composition, d'au moins un composé du type flavylium non substitué en position 3 et substitué par au moins un radical hydroxyle ou alcoxy, et tel que ceux définis ci-dessus, sous forme pure ou d'extrait végétal le contenant.
Elle a également pour objet les procédés de teinture mettant en oeuvre ces compositions.
Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Au sens de l'invention, on entend principalement par fibres kératiniques, les fibres textiles naturelles telles que la laine, les fibres kératiniques humaines telles que notamment les cheveux, les poils, les cils et les sourcils. L'invention porte plus particulièrement sur les cheveux.

Les composés de formule (I) particulièrement préférés selon la présente invention sont choisis dans le groupe de ceux pour lesquels, dans la formule (I), R₁ désigne OH ou OCH3.
On peut citer notamment parmi eux, les chlorures des composés suivants :
- 4', 5, 7-trihydroxyflavylium, communément dénommé "chlorure d'Apigénidine",
- 3', 4', 7- trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium.

Parmi ces composés, le chlorure d'Apigénidine (chlorure de 4', 5, 7-trihydroxyflavylium) et le chlorure de 3', 4', 7- trihydroxyflavylium sont encore plus particulièrement préférés.

Une forme particulière de l'invention consiste à utiliser le chlorure d'Apigénidine sous forme d'un extrait végétal, aisément préparé par extraction, et isolé, à partir de feuilles de Sorghum caudatum, ainsi que par exemple, les auteurs Kouda-Bonafos, Czyzewska, Nacro et Oehlschlager l'ont décrit et préparé dans la revue Journal of Chemical Ecology - 1994- Vol 20, N°.8 - pages 2123-2125.
Il peut aussi être extrait des tiges, des graines, ou des feuilles de Sorghum Bicolore, des pétales de Gesneria Fulgens, ainsi que des espèces Blechum Procerum et Sorgho contaminé par Colletotrichum Graminicola.

Un extrait végétal de Sorghum Caudatum titrant environ plus de 70% en poids de chlorure d'apigénidine est particulièrement préféré selon la présente invention.

Les composés du type flavylium, non substitués en position 3, et substitués par au moins un radical hydroxyle ou alcoxy, selon l'invention, peuvent être facilement obtenus par voie de synthèse, et à faible coût, notamment par la méthode bien connue de R. Robinson et D. D. Pratt [(J., Chem. Soc., 745 (1923)]. Ladite méthode implique de condenser une orthohydroxybenzaldéhyde ou ses dérivés de substitution sur une acétophénone ou ses dérivés de substitution, pour obtenir, en choisissant les substituants, les composés de formule (I) désirés.

En prenant comme exemple le chlorure d'Apigénidine (chlorure de 4', 5, 7-trihydroxyflavylium), le schéma de synthèse (i) peut être le suivant :

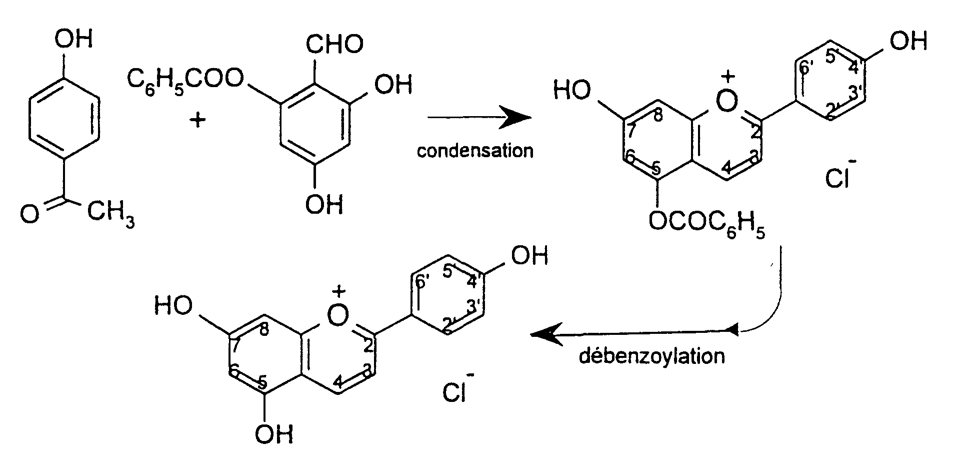

En prenant comme exemple le chlorure de 3', 4', 7- trihydroxyflavylium, le schéma de synthèse (ii) peut être le suivant :

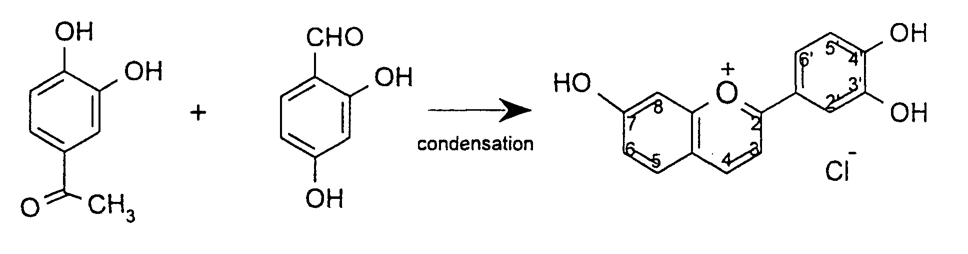

Diverses voies de synthèses, bien connues dans l'art antérieur, conduisent à l'Apigénidine.

Une méthode consiste, par exemple, à préparer, dans une première étape, la triméthylapigénidine, par condensation du 4,6-diméthoxy-2-hydroxybenzaldéhyde commercial sur la 4-méthoxyacétophénone commerciale à 0°C en milieu éther anhydre, et saturation par HCI anhydre, pour obtenir après filtration un précipité rouge-orangé de triméthylapigénidine. Dans une seconde étape, à hydrolyser la triméthylapigénidine obtenue à l'étape précédente en chlorure d'apigénidine, la réaction s'effectuant en milieu HI et phénol et AgCI en solution dans le méthanol. Une telle méthode de synthèse est décrite par R. Robinson et A. Robertson dans J., Chem., Soc. 1951- (1926) et 2196- (1927).

Une autre méthode consiste à condenser le 2, 4, 6-trihydroxybenzaldéhyde sur la 4-hydroxyacétophénone à 0°C en milieu solvant anhydre (acétate d'éthyle par exemple), et saturation par HCI anhydre, pour obtenir le chlorure d'apigénidine.
Une telle méthode est décrite par R. Robinson et A. Robertson dans J., Chem., Soc. 1528- (1928).

Une autre méthode de préparation du chlorure d'apigénidine consiste à réduire une flavone, la Naringénine, ou son dérivé triacétylé, par BH₄Na, puis à oxyder le produit obtenu par le chloranil (tétrachloro-1,4-benzoquinone).
Ladite méthode est décrite par J. G. Sweeny et G. A. lacobucci dans la revue Tetrahedron 33- 2923 et 2927- (1977).

La méthode la plus particulièrement préférée, selon la présente invention, consiste à condenser le 2,4-dihydroxy-6-benzoyl-benzaldéhyde sur la 4-hydroxyacétophénone à 0°C en milieu acétate d'éthyle anhydre, à saturer par HCI anhydre, puis à débenzoyler le produit obtenu par la soude, afin d'obtenir le chlorure d'apigénidine avec un rendement élevé, suivant le schéma (i) décrit ci-dessus.
Ladite méthode est décrite par par R. Robinson et J. C. Bell dans J., Chem., Soc. 813- (1934).

La composition tinctoriale selon l'invention peut éventuellement contenir, pour varier les nuances ou encore les enrichir en reflets, outre les composé du type flavylium non substitués en position 3 de l'invention, d'autre(s) colorants direct(s) naturels bien connus de l'état de la technique, et notamment, la 2,3-indolinedione couramment appelée isatine, des hydroxyanthraquinones, ou des benzoquinones, ou d'autres colorant(s) direct(s) de synthèse classiquement utilisés en teinture directe des cheveux, tels que par exemple, des colorants nitrés benzéniques, des nitropyridines, des colorants anthraquinoniques, des colorants mono- ou di-azoïques, triarylméthaniques, aziniques, acridiniques et xanthéniques, ou encore des colorants métallifères.

La proportion de tous ces autres colorants directs d'addition peut varier entre environ 0,05 et 10% en poids par rapport au poids total de la composition tinctoriale.

Le milieu approprié pour la teinture est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, par exemple, l'éthylèneglycol et ses éthers monométhylique, monoéthylique et monobutylique, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
On peut également utiliser des agents épaississants dans une proportion allant d'environ 0,2 à 5%.

Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des fibres kératiniques.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 4 à 11 et de préférence de 5 à 10, et pouvant être ajusté au moyen d'agents d'alcalinisation ou d'agents d'acidification ou de tampons antérieurement bien connus.
Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 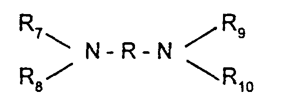 dans laquelle, R est un reste propylène éventuellement substitué par un groupement hydroxyle ou un radical alkyle en C₁-C₄; R₇ R₈, R₉ et R₁₀, simultanément ou indépendamment l'un de l'autre, représentent un atome d'hydrogène, un radical alkyle en C₁-C₄ ou hydroxyalkyle en C₁-C₄.

Les agents acidifiants sont classiquement des acides minéraux ou organiques comme par exemple les acides chlorhydrique, tartrique, citrique et phosphorique.
Parmi les tampons, on peut citer par exemple, le phosphate diacide de potassium/hydroxyde de sodium.

La composition selon l'invention, destinée à la teinture des fibres kératiniques, peut se présenter sous des formes diverses, telles que sous forme de liquide, de crème, de gel, de cataplasme, ou sous toute autre forme appropriée pour réaliser une teinture des fibres kératiniques. En particulier, elle peut être conditionnée sous pression en flacon aérosol en présence d'un agent propulseur et former une mousse.

Un autre objet de la présente invention porte sur un procédé de teinture des fibres kératiniques humaines, en particulier des cheveux, par coloration directe, consistant à laisser agir une composition tinctoriale renfermant au moins un composé du type flavylium non substitué en position 3, et substitué par au moins un radical hydroxyle ou alcoxy, sur les fibres sèches ou humides. On peut utiliser la composition selon l'invention en tant que composition non rincée, c'est-à-dire qu'après application de la composition sur les fibres, on sèche sans rinçage intermédiaire.
Dans les autres modes d'application, on laisse agir la composition sur les fibres pendant un temps de pose variant entre 3 et 60 minutes environ, de préférence entre 5 et 45 minutes environ, à une température variant entre 20°C et 50°C environ, on rince, éventuellement on lave, puis on rince à nouveau, et on sèche.

Des exemples concrets illustrant l'invention vont maintenant être donnés.

### EXEMPLE DE PREPARATION

### EXEMPLE 1 : Préparation du chlorure de 4', 5, 7-trihydroxyflavylium ou chlorure d'Apigénidine

### Première étape :

### synthèse du 2-benzoylphloroglucinaldéhyde

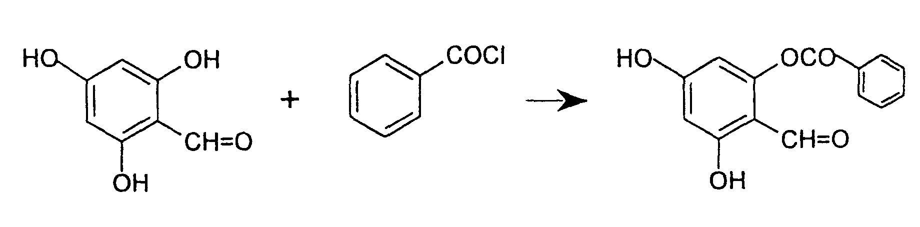

Dans un ballon tricol d'un litre, on a placé 250 ml d'eau et 15,4 g (0,1 mole) de 2,4,6-trihydroxybenzaldéhyde. On a refroidi le mélange à 7°C et on a ajouté 5,6 g (0,1 mole) de KOH dans 50 ml d'eau. On a agité fortement pendant 30 minutes puis on a refroidi à -5°C.
Au maximum de l'agitation, on a ajouté à la solution, 14 g (11 ml) de chlorure de benzoyle fraîchement distillé, en trois portions. On a agité ainsi jusqu'à disparition de l'odeur du chlorure de benzoyle, puis on a versé lentement un excès de bicarbonate dans la solution. On a essoré le précipité orange obtenu de 2-benzoylphloroglucinaldéhyde sur verre fritté, puis on a rincé au bicarbonate, puis à l'eau et enfin séché dans un dessicateur.

### Seconde étape :

### synthèse de la benzoylapigénidine

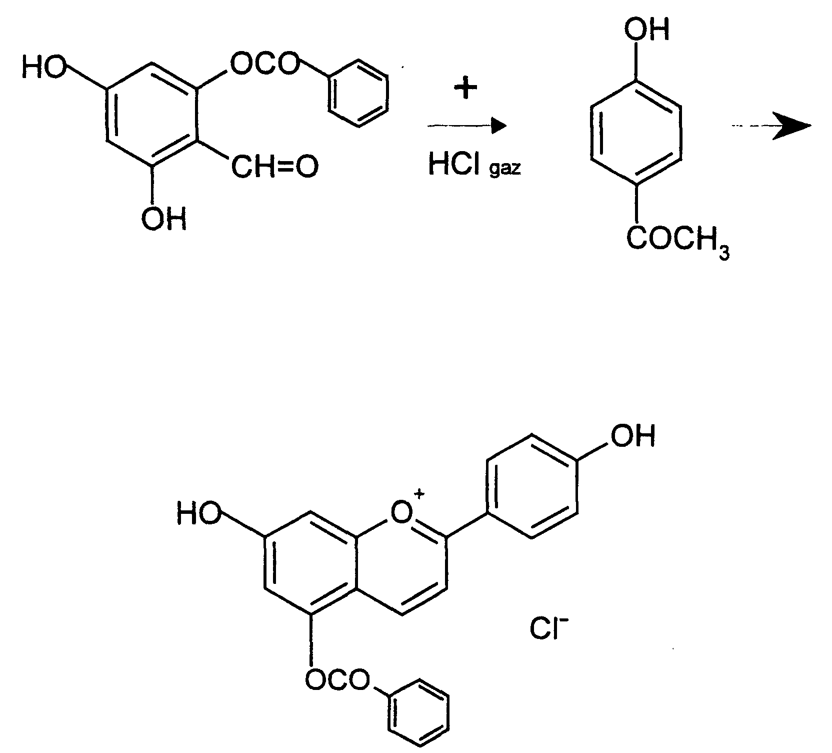

Dans un ballon bicol de 500 ml contenant 300 ml d'acétate d'éthyle anhydre (fraîchement distillé), on a placé 25,8 g (0,1 mole) de 2-benzoylphloroglucinaldéhyde obtenu à l'étape précédente avec 50 g (0,37 mole) de 4-hydroxyacétophénone. On a saturé la solution à 0°C par HCI gazeux anhydre, par barbotage pendant 35 minutes, et on a poursuivi l'agitation à 0°C pendant 78 heures. On a obtenu un précipité rouge sombre au fond du ballon, que l'on a essoré et qui était de la benzoylapigénidine - rendement : 75%.

### Troisième étape :

### synthèse du chlorure d'apigénidine

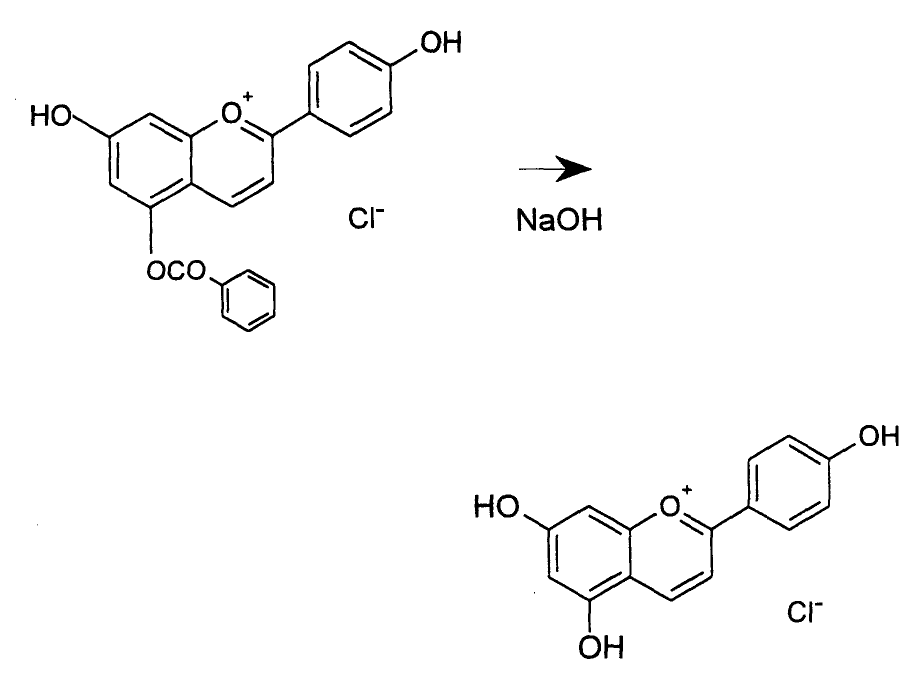

On a débenzoylé le produit obtenu à l'étape précédente en l'agitant dans une solution de soude à 10%, à température ambiante, pendant 5 heures. On a acidifié la solution par HCI concentré, on a chauffé sur bain-marie pendant 10 minutes, puis on a recristallisé le produit. On a obtenu le chlorure d'apigénidine avec 60% de rendement.

### EXEMPLE 2: Préparation du 3', 4', 7-trihydroxyflavylium

### Première étape:

### synthèse de la 3,4-dihydroxyacétophénone

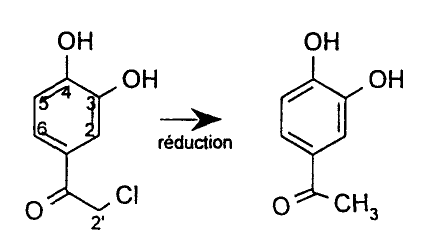

A 20 g de Zinc (préalablement décapé par de l'acide chlorhydrique 2N, lavé ensuite à l'eau jusqu'à neutralité, puis gardé à l'étuve), dans 600 ml d'un mélange de tétrahydrofurane et d'acide acétique glacial 4/1, on a ajouté 20 g de 2'-chloro-3,4-dihydroxyacétophénone. On a agité à température ambiante pendant 3 jours. On a ensuite filtré le mélange et évaporé le tétrahydrofurane. On a dissous le résidu dans de l'acétate d'éthyle, puis on a lavé à l'eau et séché sur sulfate de magnésium. On a ensuite évaporé l'acétate d'éthyle. Puis on a cristallisé le produit obtenu à partir d'un mélange acétate d'éthyle/hexane 1/1, et finalement, on a obtenu 80% d'un solide blanc par recristallisation dans de l'éthanol ou du méthanol.

### Deuxième étape :

### synthèse du chlorure de 3', 4', 7-trihydroxyflavylium.

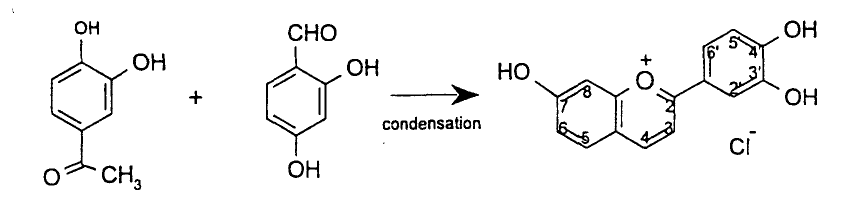

A 0°C, on a fait barboter de l'acide chlorhydrique (généré par addition goutte à goutte de H₂SO₄ sur NaCl) dans un bicol contenant 12 g (0,079 mole) de 3,4-dihydroxyacétophénone, 10,88 g (0,079 mole) de 2,4-dihydroxybenzaldéhyde et 100ml d'acétate d'éthyle préalablement distillé. Le produit a commencé à précipiter au bout de 30 minutes. On a laissé barboter encore pendant 1 heure, puis on a arrêté la réaction. On a gardé la solution à -18°C durant 3 jours, puis on l'a filtrée. On a évaporé le filtrat, et on l'a repris dans de l'éther; le produit a précipité. On l'a séparé de l'éther, dissous dans du méthanol chaud, puis on lui a ajouté lentement un volume équivalent d'acétone. On a mis la solution au réfrigérateur, le produit a commencé à précipiter. Après filtration le filtrat a été évaporé, dissous dans un minimum de méthanol, puis on a ajouté de l'éther jusqu'à ce qu'un précipité apparaisse. L'opération a été ainsi répétée plusieurs fois jusqu'à ce qu'il n'apparaisse plus de précipité.
On a vérifié la pureté du précipité de chlorure de 3', 4',7-trihydroxyflavylium ainsi obtenu avec 75% de rendement, en HPLC (chromatographie liquide à haute performance).

### EXEMPLES D'APPLICATION

### EXEMPLE 3 :

On a préparé la composition de teinture suivante :

| | |
|---|---|
| Chlorure de 3', 4', 7-trihydroxyflavylium préparé à l'exemple 2 | 0,29 g |
| Hydroxyéthylcellulose | 2,30 g |
| Acide citrique | 1,40 g |
| Solution aqueuse de NaOH qs pH | 2,65 |
| Eau déminéralisée qsp | 100 g |

On a appliqué la composition ci-dessus sur des mèches de cheveux gris naturels et également sur des mèches de cheveux gris permanentés, à 90% de blancs, à raison de 2 grammes de composition par gramme de cheveux. On a laissé poser pendant 30 minutes à température ambiante. Après rinçage à l'eau courante et séchage, les cheveux ont été teints dans une nuance rouge acajou bien résistante à la lumière, aux shampooings et aux intempéries.

### EXEMPLE 4:

On a préparé la composition de teinture suivante :

| | |
|---|---|
| Chlorure d'apigénidine préparé à l'exemple 1 | 1,00 g |
| Hydroxyéthylcellulose | 2,66 g |
| Alcool éthylique | 16,00 g |
| Solution aqueuse de NaOH qs pH... | 9,5 |
| Eau déminéralisée qsp | 100 g |

On a appliqué la composition ci-dessus sur des mèches de cheveux gris naturels et également sur des mèches de cheveux gris permanentés, à 90% de blancs, à raison de 2 grammes de composition par gramme de cheveux. On a laissé poser pendant 30 minutes à 45°C. Après rinçage à l'eau courante et séchage, les cheveux ont été teints dans une nuance rouge acajou bien résistante aux shampooings, à la transpiration et à la lumière.

### EXEMPLE 5 :

On a préparé la composition de teinture suivante :

| | |
|---|---|
| Chlorure d'apigénidine préparé à l'exemple 1 | 1,00 g |
| Hydroxyéthylcellulose | 2,66 g |
| Alcool éthylique | 16,00 g |
| Solution aqueuse de HCl qs pH | 5,5 |
| Eau déminéralisée qsp | 100 g |

On a appliqué la composition ci-dessus sur des mèches de cheveux gris naturels et également sur des mèches de cheveux gris permanentés, à 90% de blancs, à raison de 2 grammes de composition par gramme de cheveux. On a laissé poser pendant 30 minutes à 45°C. Après rinçage à l'eau courante et séchage, les cheveux ont été teints dans une nuance rouge acajou bien résistante aux shampooings, à la transpiration et à la lumière.

## Revendications

1. Utilisation de composés du type flavylium, non substitués en position 3, et substitués par au moins un radical hydroxyle ou alcoxy, sous forme pure ou d'extraits végétaux les contenant, à titre de colorants, dans une, ou pour la préparation d'une, composition destinée à la teinture des fibres kératiniques.

2. Utilisation selon la revendication 1, caractérisée par le fait qu'il s'agit de fibres kératiniques humaines et notamment les cheveux.

3. Utilisation selon l'une quelconque des revendications précédentes, caractérisée par le fait que le composé du type flavylium est substitué en position 4' par un radical hydroxy, alcoxy ou hydroxyalkyle.

4. Utilisation selon la revendication 3, caractérisée par le fait que le composé du type flavylium est un composé de formule (I) suivante : 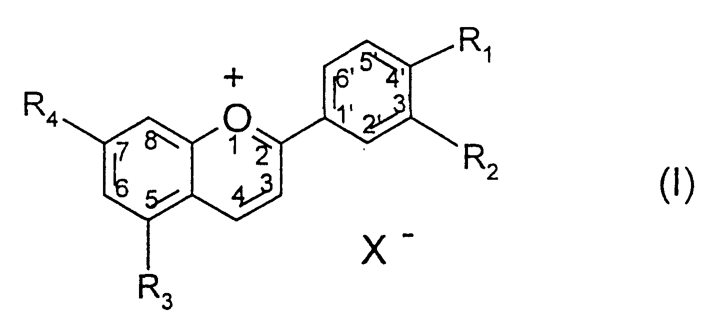 formule (I) dans laquelle:
- **R**_{**1**} désigne un radical OH ou alcoxy en C₁-C₈, linéaire ou ramifié, saturé ou insaturé,
- **R**_{**2**}**, R**_{**3**}**, R**_{**4**}**,** identiques ou différents, désignent H ou R₁,
étant entendu qu'au moins un des radicaux R₁ à R₄ désigne OH,
- X⁻ est un anion désignant de préférence un halogène ou un radical: 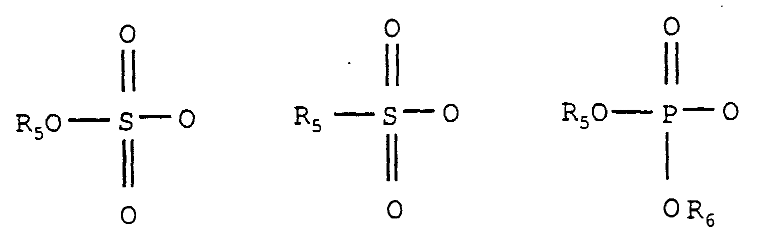 dans lesquels R₅ et R₆ sont des radicaux alkyles en C₁-C₈, linéaires ou ramifiés, saturés ou insaturés.

5. Utilisation selon la revendication 4, caractérisée par le fait que dans la formule (I), R₁ désigne OH ou OCH₃.

6. Utilisation selon la revendication 5, caractérisée par le fait que les composés de formule (I) sont choisis dans le groupe formé par les chlorures de :
- 4', 5, 7-trihydroxyflavylium,
- 3', 4', 7-trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium.

7. Utilisation selon la revendication 6, caractérisée par le fait qu'il s'agit du chlorure de 4', 5, 7-trihydroxyflavylium, sous forme pure.

8. Utilisation selon la revendication 6, caractérisée par le fait qu'il s'agit du chlorure de 4', 5, 7-trihydroxyflavylium, sous forme d'extrait végétal.

9. Utilisation selon la revendication 8, caractérisée par le fait que l'extrait végétal est un extrait de Sorghum Caudatum.

10. Utilisation selon la revendication 9, caractérisée par le fait que l'extrait titre plus de 70% de chlorure de 4', 5, 7-trihydroxyflavylium.

11. Composition de teinture des fibres kératiniques, comprenant, dans un milieu approprié pour la teinture, 0,05 à 20% en poids par rapport au poids total de la composition d'au moins un composé du type flavylium non substitué en position 3, et substitué par au moins un radical hydroxyle ou alcoxy, sous forme pure ou d'extrait végétal le contenant.

12. Composition selon la revendication 11, caractérisée par le fait que ledit composé du type flavylium représente 0,1 à 10% en poids par rapport au poids total de la composition.

13. Composition selon la revendication 11 ou 12, caractérisée par le fait qu'il s'agit de fibres kératiniques humaines et notamment les cheveux.

14. Composition selon l'une quelconque des revendications 11 à 13, caractérisée par le fait que le composé du type flavylium est substitué en position 4' par un radical hydroxy, alcoxy ou hydroxyalkyle.

15. Composition selon la revendication 14, caractérisée par le fait que le composé du type flavylium est un composé de formule (I) suivante : 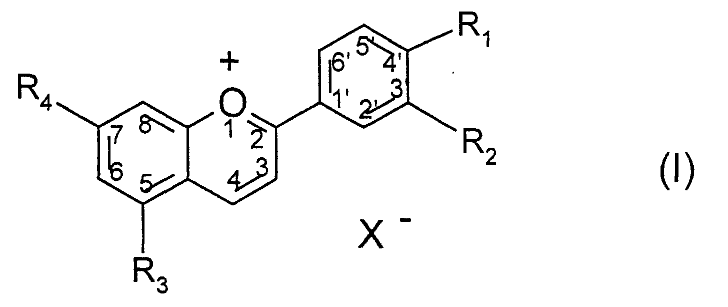 formule (I) dans laquelle :
- **R**_{**1**} désigne un radical OH ou alcoxy en C₁-C₈, linéaire ou ramifié, saturé ou insaturé,
- **R**_{**2**}**, R**_{**3**}**, R**_{**4**}**,** identiques ou différents, désignent H ou R₁,
étant entendu qu'au moins un des radicaux R₁ à R₄ désigne OH,
- X⁻ est un anion désignant de préférence un halogène ou un radical : 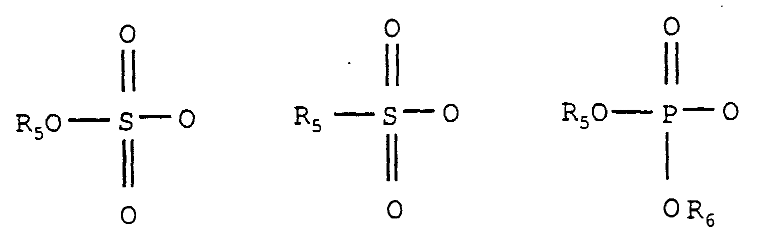 dans lesquels R₅ et R₆ sont des radicaux alkyles en C₁-C₈, linéaires ou ramifiés, saturés ou insaturés.

16. Composition selon la revendication 15, caractérisée par le fait que dans la formule (I), R₁ désigne OH ou OCH₃.

17. Composition selon la revendication 16, caractérisée par le fait que les composés de formule (I) sont choisis dans le groupe formé par les chlorures de :
- 4', 5, 7-trihydroxyflavylium,
- 3', 4', 7-trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium.

18. Composition selon la revendication 17, caractérisée par le fait qu'il s'agit du chlorure de 4', 5, 7-trihydroxyflavylium, sous forme pure.

19. Composition selon la revendication 17, caractérisée par le fait qu'il s'agit du chlorure de 4', 5, 7-trihydroxyflavylium, sous forme d'extrait végétal.

20. Composition selon la revendication 19, caractérisée par le fait que l'extrait végétal est un extrait de Sorghum Caudatum.

21. Composition selon la revendication 20, caractérisée par le fait que l'extrait titre plus de 70% de chlorure de 4', 5, 7-trihydroxyflavylium.

22. Composition selon l'une quelconque des revendications 11 à 21, caractérisée par le fait qu'elle a un pH compris entre 4 et 11.

23. Procédé de teinture des fibres kératiniques humaines, en particulier des cheveux, par teinture directe, caractérisé par le fait qu'on applique une composition tinctoriale renfermant au moins un composé du type flavylium non substitué en position 3, tel que défini à l'une quelconque des revendications 1 à 10, sur les fibres kératiniques sèches ou humides, et qu'après avoir éventuellement laissé agir la composition sur les fibres pendant un temps de pose allant de 3 à 60 minutes, à une température comprise entre 20°C et 50°C, on sèche ces fibres, après un rinçage éventuel.

## Patentansprüche

1. Verwendung von Verbindungen vom Flavyliumtyp, die in 3-Stellung nicht substituiert sind und die mit mindestens einer Hydroxy- oder Alkoxygruppe substituiert sind, in reiner Form oder in Form von Pflanzenextrakten, die diese Verbindungen enthalten, als Farbstoffe in Zusammensetzungen, die zum Färben von Keratinfasern vorgesehen sind, oder zur Herstellung dieser Zusammensetzungen.

2. Verwendung nach Anspruch 1, dadurch gekennzeichnet, daß es sich um menschliche Keratinfasern und insbesondere Haare handelt.

3. Verwendung nach einem der vorhergehenden Ansprüche, dadurch gekennzeichnet, daß die Verbindung vom Flavyliumtyp in 4'-Stellung mit Hydroxy, Alkoxy oder Hydroxyalkyl substituiert ist.

4. Verwendung nach Anspruch 3, dadurch gekennzeichnet, daß die Verbindung vom Flavyliumtyp eine Verbindung der folgenden Formel (I) ist: 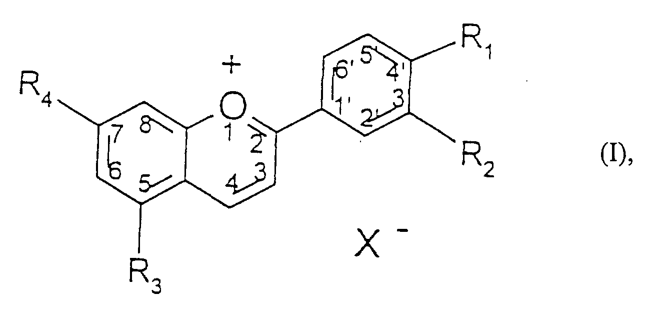 wobei in der Formel (I) bedeuten:
- R₁ OH oder eine geradkettige oder verzweigte, gesättigte oder ungesättigte C₁₋₈-Alkoxygruppe,
- R₂, R₃ und R₄, die identisch oder voneinander verschieden sind, H oder R₁,
- X⁻ ein Anion, und vorzugsweise ein Halogenid oder eine Gruppe: 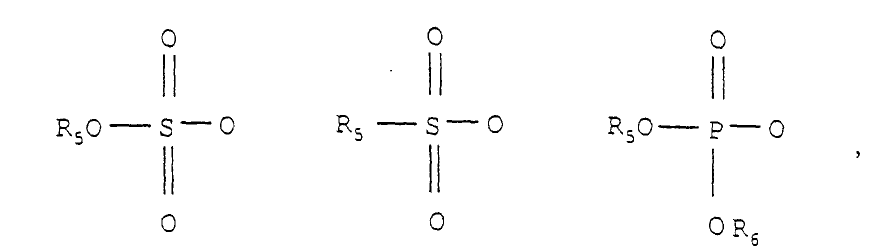 worin R₅ und R₆ geradkettige oder verzweigte, gesättigte oder ungesättigte C₁₋₈-Alkylgruppen bedeuten.

5. Verwendung nach Anspruch 4, dadurch gekennzeichnet, daß in der Formel (I) R₁ OH oder OCH₃ bedeutet.

6. Verwendung nach Anspruch 5, dadurch gekennzeichnet, daß die Verbindungen der Formel (I) unter den Chloriden der folgenden Verbindungen ausgewählt sind:
- 4',5,7-Trihydroxyflavylium,
- 3',4',7-Trihydroxyflavylium,
- 4'-Hydroxyflavylium,
- 4',7-Dihydroxyflavylium,
- 3',4'-Dihydroxyflavylium,
- 3',4'-Dihydroxy-7-methoxyflavylium.

7. Verwendung nach Anspruch 6, dadurch gekennzeichnet, daß es sich um 4',5,7-Trihydroxyflavyliumchlorid in reiner Form handelt.

8. Verwendung nach Anspruch 6, dadurch gekennzeichnet, daß es sich um 4',5,7-Trihydroxyflavyliumchlorid in Form eines Pflanzenextrakts handelt.

9. Verwendung nach Anspruch 8, dadurch gekennzeichnet, daß der Pflanzenextrakt ein Extrakt von Sorghum caudatum ist.

10. Verwendung nach Anspruch 9, dadurch gekennzeichnet, daß der Extrakt mehr als 70 % 4',5,7-Trihydroxyflavyliumchlorid enthält.

11. Zusammensetzung zum Färben von Keratinfasern, die in einem zum Färben geeigneten Medium in reiner Form oder als Pflanzenextrakt, der diese Verbindung enthält, 0,05 bis 20 Gew.-% mindestens einer Verbindung vom Flavyliumtyp, die in 3-Stellung nicht substituiert ist und die mit mindestens einer Hydroxy- oder Alkoxygruppe substituiert ist, bezogen auf das Gesamtgewicht der Zusammensetzung, enthält.

12. Zusammensetzung nach Anspruch 11, dadurch gekennzeichnet, daß die Verbindung vom Flavyliumtyp 0,1 bis 10 Gew.-%, bezogen auf das Gesamtgewicht der Zusammensetzung, ausmacht.

13. Zusammensetzung nach Anspruch 11 oder 12, dadurch gekennzeichnet, daß es sich um menschliche Keratinfasern und insbesondere das Haar handelt.

14. Zusammensetzung nach einem der Ansprüche 11 bis 13, dadurch gekennzeichnet, daß die Verbindung vom Flavyliumtyp in 4'-Stellung mit Hydroxy, Alkoxy oder Hydroxyalkyl substituiert ist.

15. Zusammensetzung nach Anspruch 14, dadurch gekennzeichnet, daß die Verbindung vom Flavyliumtyp eine Verbindung der folgenden Formel (I) ist: 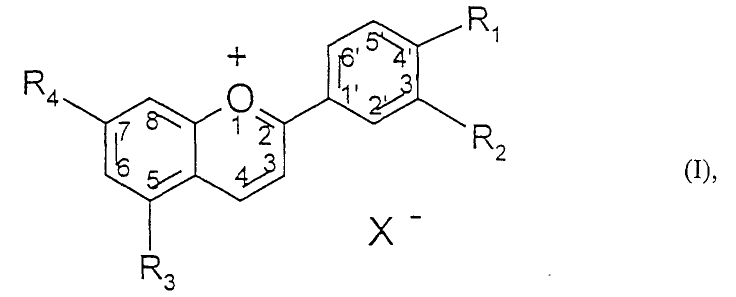 wobei in der Formel (I) bedeuten:
- R₁ OH oder eine geradkettige oder verzweigte, gesättigte oder ungesättigte C₁₋₈-Alkoxygruppe,
- R₂, R₃ und R₄, die identisch oder voneinander verschieden sind, H oder R₁,
- mit der Maßgabe, daß mindestens eine der Gruppen R₁ bis R₄ OH bedeutet,
- X⁻ ein Anion, und vorzugsweise ein Halogenid oder eine Gruppe:

16. Zusammensetzung nach Anspruch 15, dadurch gekennzeichnet, daß in der Formel (I) R₁ OH oder OCH₃ bedeutet.

17. Zusammensetzung nach Anspruch 16, dadurch gekennzeichnet, daß die Verbindungen der Formel (I) unter den Chloriden der folgenden Verbindungen ausgewählt sind:
- 4',5,7-Trihydroxyflavylium,
- 3',4',7-Trihydroxyflavylium,
- 4'-Hydroxyflavylium,
- 4',7-Dihydroxyflavylium,
- 3',4'-Dihydroxyflavylium,
- 3',4'-Dihydroxy-7-methoxyflavylium.

18. Zusammensetzung nach Anspruch 17, dadurch gekennzeichnet, daß es sich um das 4',5,7-Trihydroxyflavyliumchlorid in reiner Form handelt.

19. Zusammensetzung nach Anspruch 17, dadurch gekennzeichnet, daß es sich um das 4',5,7-Trihydroxyflavyliumchlorid in Form eines Pflanzenextrakts handelt.

20. Zusammensetzung nach Anspruch 19, dadurch gekennzeichnet, daß der Pflanzenextrakt ein Extrakt von Sorghum caudatum ist.

21. Zusammensetzung nach Anspruch 20, dadurch gekennzeichnet, daß der Extrakt mehr als 70 % 4',5,7-Trihydroxyflavyliumchlorid enthält.

22. Zusammensetzung nach einem der Ansprüche 11 bis 21, dadurch gekennzeichnet, daß sie einen pH-Wert von 4 bis 11 aufweist.

23. Verfahren zum Färben von menschlichen Keratinfasern und insbesondere dem Haar durch direktes Färben, dadurch gekennzeichnet, daß eine Färbemittelzusammensetzung auf die trockenen oder feuchten Keratinfasern aufgebracht wird, die mindestens eine in 3-Stellung nicht substituierte Verbindung vom Flavyliumtyp nach einem der Ansprüche 1 bis 10 enthält, worauf die Zusammensetzung gegebenenfalls während einer Zeitspanne von 3 bis 60 min bei einer Temperatur von 20 bis 50 °C auf die Fasern einwirken gelassen wird und die Fasern dann gegebenenfalls nach einem Spülschritt getrocknet werden.

## Claims

1. Use of flavylium-type compounds non-substituted in position 3, and substituted by at least one hydroxyl or alkoxy radical, in pure form or in the form of plant extracts containing them, as colouring agents, in or for the preparation of a composition for dyeing keratinous fibres.

2. Use according to Claim 1, characterized in that it involves human keratinous fibres and in particular hair.

3. Use according to any one of the preceding claims, characterized in that the flavylium-type compound is substituted in position 4' by a hydroxyl, alkoxy or hydroxyalkyl radical.

4. Use according to Claim 3, characterized in that the flavylium-type compound is a compound of the following formula (I): 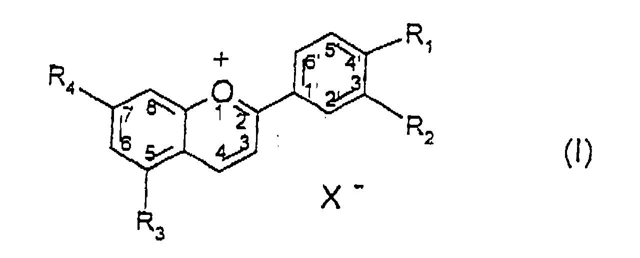 in which formula (I):
- **R**_{**1**} denotes an OH radical or a saturated or unsaturated, linear or branched C₁-C₈ alkoxy radical,
- **R**_{**2**}**, R**_{**3**} and **R**_{**4**}**,** which are identical or different, denote H or R₁,
it being understood that at least one of the radicals R₁ to R₄ denotes OH,
- X⁻ is an anion preferably denoting a halogen or a radical: 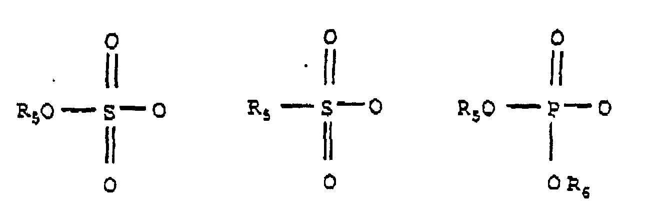 in which R₅ and R₆ are saturated or unsaturated, linear or branched C₁-C₈ alkyl radicals.

5. Use according to Claim 4, characterized in that in formula (I), R₁ denotes OH or OCH₃.

6. Use according to Claim 5, characterized in that the compounds of formula (I) are chosen from the group consisting of the chlorides of:
- 4',5,7-trihydroxyflavylium,
- 3',4',7-trihydroxyflavylium,
- 4'-hydroxyflavylium,
- 4',7-dihydroxyflavylium,
- 3',4'-dihydroxyflavylium,
- 3',4'-dihydroxy-7-methoxyflavylium.

7. Use according to Claim 6, characterized in that it involves 4',5,7-trihydroxyflavylium chloride in pure form.

8. Use according to Claim 6, characterized in that it involves 4',5,7-trihydroxyflavylium chloride in the form of a plant extract.

9. Use according to Claim 8, characterized in that the plant extract is an extract of Sorghum caudatum,

10. Use according to Claim 9, characterized in that the extract has a titre greater than 70% of 4',5,7-trihydroxyflavylium chloride.

11. Composition for dyeing keratinous fibres, comprising, in an appropriate medium for dyeing, 0.05 to 20% by weight, relative to the total weight of the composition, of at least one flavylium-type compound non-substituted in position 3, and substituted by at least one hydroxyl or alkoxy radical, in pure form or in the form of a plant extract containing it.

12. Composition according to Claim 11, characterized in that the said compound of the flavylium type represents 0.1 to 10% by weight relative to the total weight of the composition.

13. Composition according to Claim 11 or 12, characterized in that it involves human keratinous fibres and in particular hair.

14. Composition according to any one of Claims 11 to 13, characterized in that the flavylium-type compound is substituted in position 4' by a hydroxyl, alkoxy or hydroxyalkyl radical.

15. Composition according to Claim 14, characterized in that the flavylium-type compound is a compound of the following formula (I): 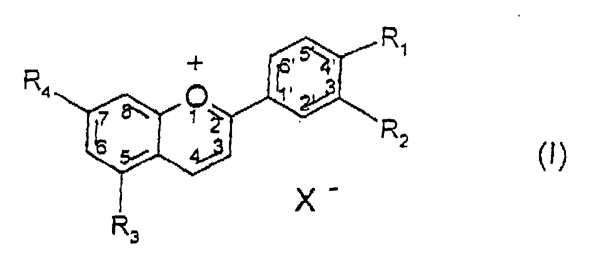 in which formula (I):
- **R**_{**1**} denotes an OH radical or a saturated or unsaturated, linear or branched C₁-C₈ alkoxy radical,
- **R**_{**2**}**, R**_{**3**} **and R**_{**4**}**,** which are identical or different, denote H or R₁,
it being understood that at least one of the radicals R₁ to R₄ denotes OH,
- X⁻ is an anion preferably denoting a halogen or a radical: 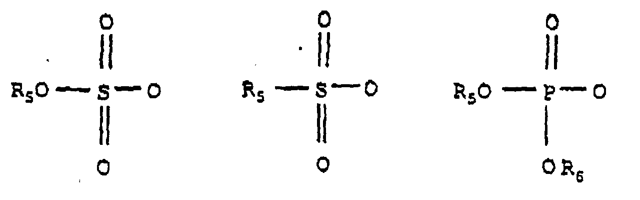 in which R₅ and R₆ are saturated or unsaturated, linear or branched C₁-C₆ alkyl radicals.

16. Composition according to Claim 15, characterized in that in formula (I), R₁ denotes OH or OCH₃.

17. Composition according to Claim 16, characterized in that the compounds of formula (I) are chosen from the group consisting of the chlorides of:
- 4',5,7-trihydroxyflavylium,
- 3',4',7-trihydroxyflavylium,
- 4'-hydroxyflavylium,
- 4',7-dihydroxyflavylium,
- 3',4'-dihydroxyflavylium,
- 3',4'-dihydroxy-7-methoxyflavylium.

18. Composition according to Claim 17, characterized in that it involves 4',5,7-trihydroxyflavylium chloride in pure form.

19. Composition according to Claim 17, characterized in that it involves 4',5,7-trihydroxyflavylium chloride in the form of a plant extract,

20. Composition according to Claim 19, characterized in that the plant extract is an extract of Sorghum caudatum.

21. Composition according to Claim 20, characterized in that the extract has a titre greater than 70% of 4',5,7-trihydroxyflavylium chloride.

22. Composition according to any one of Claims 11 to 21, characterized in that it has a pH of between 4 and 11.

23. Method of dyeing human keratinous fibres, in particular hair, by direct dyeing, characterized in that a dyeing composition containing at least one flavylium-type compound non-substituted in position 3, as defined in any one of Claims 1 to 10, is applied to the dry or wet keratinous fibres, and in that after having optionally allowed the composition to act on the fibres for an exposure time ranging from 3 to 60 minutes, at a temperature of between 20°C and 50°C, these fibres are dried, after optional rinsing.
